Europäisches Patentamt

European Patent Office

Office européen des brevets

(19)

(11) Veröffentlichungsnummer: **0 233 539 B1**

(12) **EUROPÄISCHE PATENTSCHRIFT**

(45) Veröffentlichungstag der Patentschrift:
26.09.90

(51) Int. Cl.⁵: **G01N 3/18**

(21) Anmeldenummer: **87101435.3**

(22) Anmeldetag: **03.02.87**

(54) **Materialprüfvorrichtung.**

(30) Priorität: **18.02.86 DE 3605154**

(43) Veröffentlichungstag der Anmeldung:
**26.08.87 Patentblatt 87/35**

(45) Bekanntmachung des Hinweises auf die Patenterteilung:
**26.09.90 Patentblatt 90/39**

(84) Benannte Vertragsstaaten:
**AT CH FR GB LI SE**

(56) Entgegenhaltungen:
**CH-A- 378 069**

**INDUSTRIAL LABORATORY, Band 49, Nr. 5, Mai 1983, Seiten 541-544, Plenum Publishing Corp., New York, US; A.P. KORCHAGIN et al.: "Apparatus for the investigation of mechanical properties of metals in gaseous hydrogen at high temperatures and pressures" parenchymal and non-parenchymal cells of rat liver", & BIOCHIM. BIOPHYS. ACTA 1985, 842(1), 12-21 000**
**REVIEW OF SCIENTIFIC INSTRUMENTS, Band 49, Nr. 1, Januar 1978, Seiten 50,51, American Institut of Physics, US; K. YOKOGAWA et al.: "Pressure vessel for tensile testing in high-pressure gases at elevated temperatures"**

(73) Patentinhaber: **MTU MOTOREN- UND TURBINEN-UNION MÜNCHEN GMBH, Dachauer Strasse 665 Postfach 50 06 40, D-8000 München 50(DE)**

(72) Erfinder: **Radziwill, Eberhard Günter, Rua Moacir Miguel da Silva 366 Condominio, casa 70, Jardim Bonfiglioli CEP 05595 - Sao Paulo(BR)**

## Beschreibung

Die Erfindung betrifft eine Materialprüfvorrichtung für Zeitstandsprüfungen an Probekörpern aus metallischen oder keramischen Werkstoffen, bei der der Probekörper beidendig eingespannt ist und unter erhöhter Temperatur auf Zug belastbar ist, wobei ein Probenende mit einer motorisch getriebenen Spindel zum Zweck der Be- und Entlastung gekoppelt ist, und Dehnung oder Kontraktion mittels einer Meßvorrichtung mit Dehnungsaufnehmern abfühlbar ist.

In "Industrial Laboratory", Band 49, Nr. 5, 1983, Seite 541 – 544 ist eine gattungsgemäße Materialprüfvorrichtung offenbart, bei der axiale Lasten aufbringbar sind sowie eine automatische Versuchsdurchführung erzielbar ist.

Diese Anordnung dient dazu, Proben bis zum Bruch zu belasten.

"Review of Scientific Instruments", Band 49, Nr. 1, 1978, Seite 50, 51 betrifft eine Materialprüfvorrichtung, mittels der eine Probe unter hohem Druck bei hoher Temperatur belastbar ist. Dabei ist eine Kompensationsvorrichtung zum Ausgleich der Axialdruckkräfte vorgesehen, die innerhalb des Druckbehälters auf die Probe und die Spannköpfe wirken. Dies geschieht, um definierte Lastverhältnisse zu erhalten.

Die CH-A 378 069 befaßt sich mit einer Thermokriech-Meßvorrichtung, mit der zwei parallel angeordnete Proben erhitzt werden und gleichzeitig eine der Proben auf Zug belastet wird. Die dadurch bedingte Dehnung wird einerseits über eine Kontaktsteuerung durch eine Verminderung der Zuglast ausgeglichen, andererseits ist diese direkt meßbar.

Derartige Materialprüfvorrichtungen werden sowohl für einzelne Prüfungen als auch als Universalprüfmaschinen geliefert, um vorbereitete Proben entweder für eine bestimmte Zeitdauer (auf Zeitstandsanlage) oder als statistische Lebensdauerprüfanlage zu untersuchen, d. h., bis zur Zerstörung des Probestücks wie Bruch, Riß o. ä.. Bevorzugt werden jedoch Zeit- oder Dehnungsversuche, sogenannte Kriechversuche nach DIN 50 118 oder in ähnlicher oder abgewandelter Form.

Diese Prüfvorrichtungen weisen in einem Sockel eine motorisch angetriebene Spindel auf, die mit der Dehnungsmeßeinrichtung starr verbunden ist. Dabei ist die Probe in einem Ofen befindlich, der seinerseits in einem Gestell angeordnet ist, mit wenigstens zwei Säulen und wenigstens einer Traverse derart, daß der Probenhalter an einem zweiseitigen Hebel aufgehängt ist, an dessen, der Probe abgekehrtem Ende ein Belastungsgewicht eingehängt ist.

Ein Nachteil dieser vorbekannten Anordnungen besteht darin, daß zum einen bei Probenbruch und zum zweiten bei Prüfungsbeginn und -ende starke Belastungen auf die Prüfvorrichtung bzw. die Probe wirken. So sind bei Probenbruch starke Kräfte plötzlich abzufangen, was insbesondere bei Hochtemperaturversuchen zu starken Beanspruchungen der schon thermisch belasteten Bauteile führt.

Bei Versuchsbeginn und nach Versuchsende erfolgt eine Temperaturveränderung der Probe und Einspannelemente, was zu erheblichen Thermodehnungen führt.

Wegen der Wärmedehnung bei Aufheizen bzw. Kontraktion bei Abkühlung muß bei Verzuchsanfang bzw. Versuchsende Bedienungspersonal anwesend sein, um die Anlage ein- bzw. abzuschalten, insbesondere den Ofen und/oder den Antrieb an- bzw. abzukoppeln.

Hierdurch wird die Verwendung bekannter Vorrichtungen auf vorgegebene Zeiten (Arbeitszeiten) begrenzt. Erfolgt ausnahmsweise keine rechtzeitige Überwachung, z. B. am Versuchsende, so besteht die Gefahr einer Zerstörung von Maschinenteilen bzw. Teilen der Meßeinrichtung und die der Probe.

Aufgabe der Erfindung ist es, eine Materialprüfvorrichtung der in Rede stehenden Art zu schaffen, bei der einerseits plötzliche Proberisse ohne Auswirkung auf die Vorrichtung bleiben, andererseits nach einer automatischen Abschaltung der Vorrichtung ein Ausgleich der Thermodehnungen (Abkühlkontraktionen) erfolgen kann.

Gelöst wird diese Aufgabe durch die im Hauptanspruch aufgeführten Merkmale. Weitere Merkmale sind den abhängigen Ansprüchen zu entnehmen.

Der wesentliche Vorteil, der mit der Erfindung erreicht wird, ist darin zu sehen, daß die Prüfvorrichtung rund um die Uhr gefahrlos betrieben werden kann. Auch dehnungs- und zeitgesteuerte Versuche, d. h. Versuche über eine vorbestimmte Dauer, lassen sich nunmehr gefahrlos durchführen. Nach Beendigung der Sollzeit fährt dann die Antriebsspindel in Entlastungsrichtung, schaltet mittels Endschalter ab nach dem tatsächlichen Entlasten der Probe und Ofen und Zeitwerk sind abgeschaltet, ebenso der Antrieb, der erneut weiterfährt, bis genügend Weg für die Kontraktion der Probe und des Meßaufbaus vorhanden ist. Bei Versuchen, die mit einer (vorzeitigen) Zerstörung der Probe enden, ist sichergestellt, daß der Endschalter ebenfalls tatsächlich betätigt wird und die oben genannten Abschaltungen erfolgen. Eine Gefahr der Zerstörung oder Beschädigung von Maschinenteilen oder Teilen der Meßeinrichtung besteht nicht mehr. Eine Zerstörung soll der Probe jedoch nur ausnahmsweise erfolgen. In der Regel soll auf der Vorrichtung eine zerstörungsfreie Materialprüfung durchgeführt werden.

Bei der Erfindung ist weiterhin vorteilhaft eine Bewegungsdämpfungseinrichtung für den mit der Probe verbundenen Kolben, der in einen Zylinder hineinbewegbar ist und somit verhindert, daß teure Quarzglasstäbe einer Dehnungsmeßeinrichtung wie inkrementale Linearmeßsysteme einem Bruch ausgeliefert sind.

Weitere Vorteile der Erfindung sind der Beschreibung und Zeichnung eines Ausführungsbeispiels zu entnehmen, das den besten Weg zur gewerblichen Anwendung der Erfindung aufzeigt.

Die Zeichnungen zeigen das Ausführungsbeispiel der Erfindung rein schematisch in:

Fig. 1 eine Vorderansicht der Materialprüfvorrichtung

Fig. 2 eine Seitenansicht der Materialprüfeinrichtung

Fig. 3 a), b) und c) Hysterese-Schalt- und Dämpfungsvorrichtung mit dem Zylinder, in den der Kolben sich hineinbewegt sowie den Endschalter mit seinem in verschiedenen Positionen verharrenden Betätigungselement.

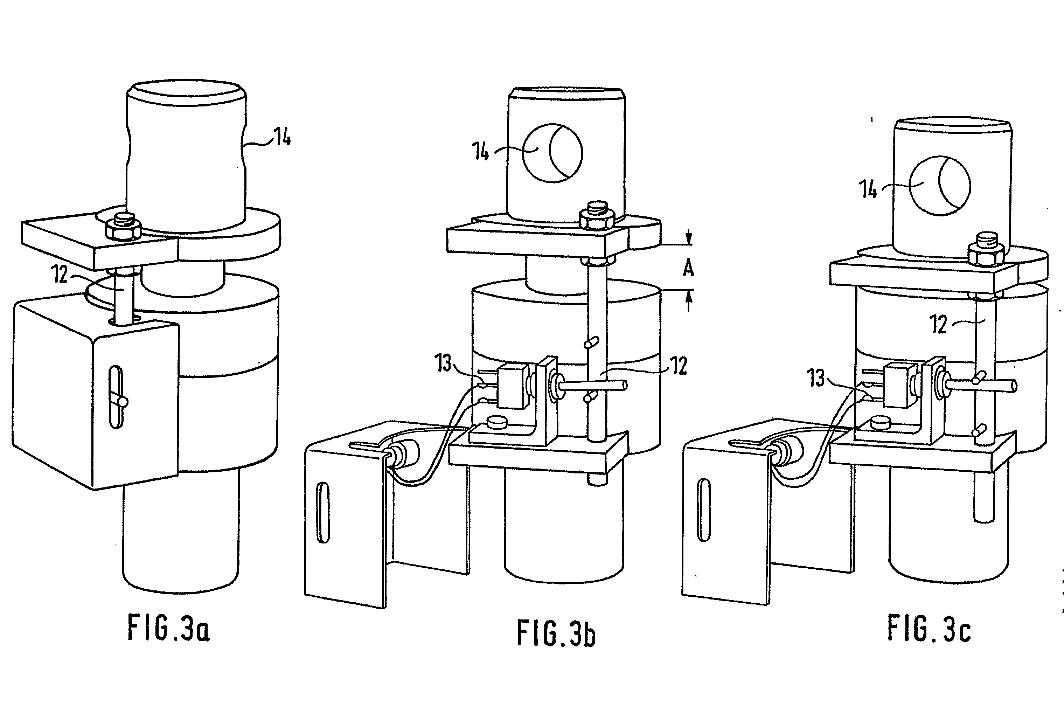

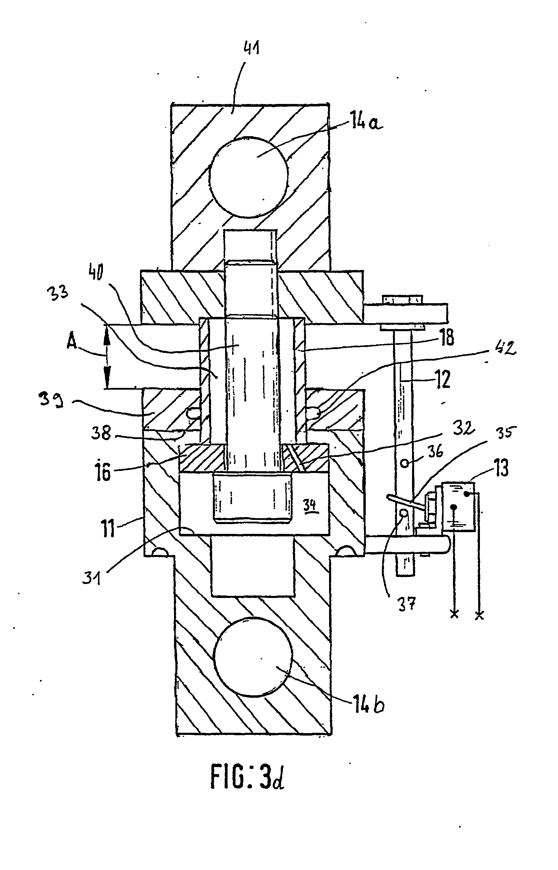

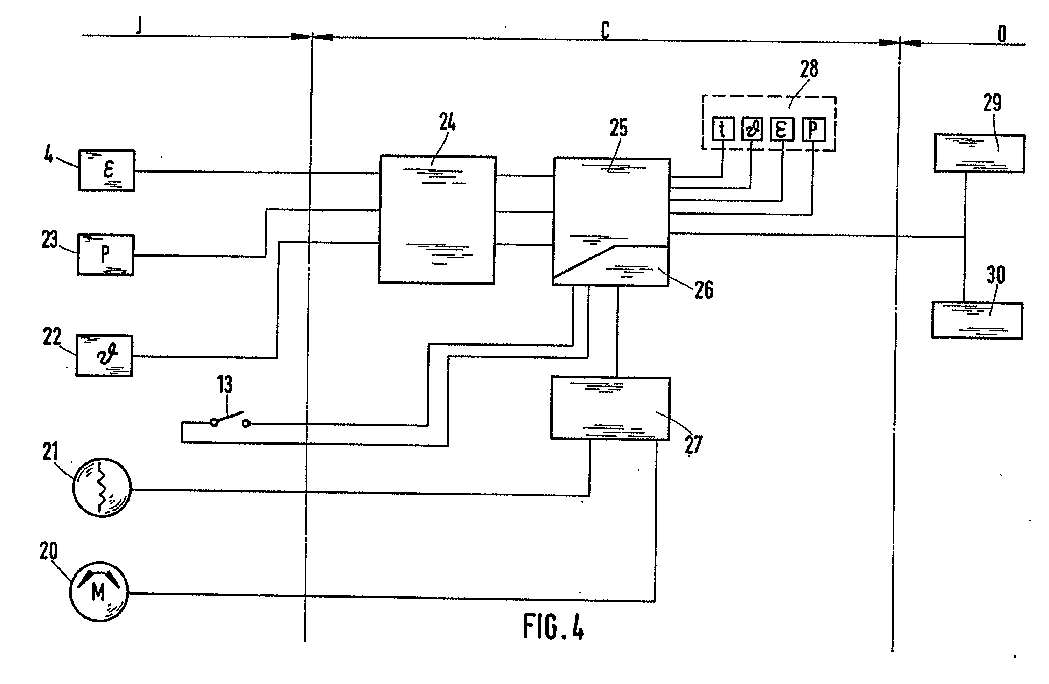

Fig. 3 d) Schnittbild zu 3 a) – 3 c) Fig. 4

zeigt eine Blockschaltbild der HystereseSchalt- und Dämpfungsvorrichtung.

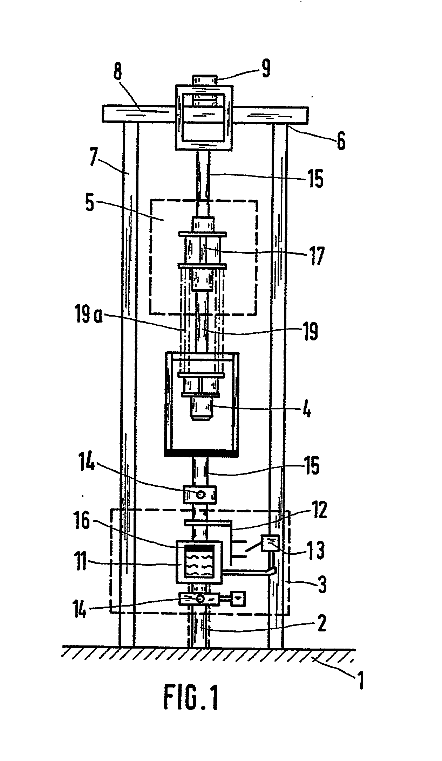

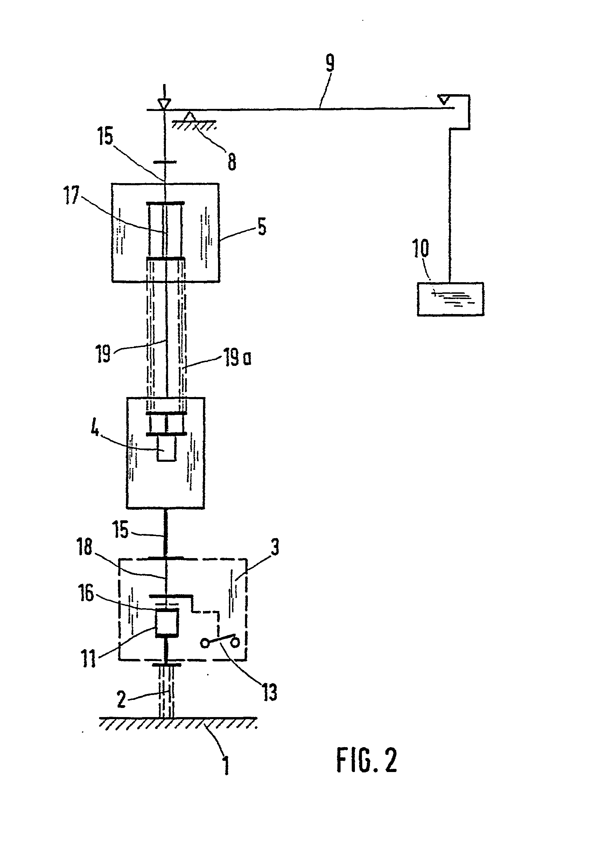

Wie Figur 1 zeigt, weist die Materialprüfvorrichtung einen Maschinensockel 1 mit dem Antrieb z.B. einer Spindel 2 eine Dehnungsausgleichsvorrichtung (Hysterese-Schalt- und Dämpfungsvorrichtung) 3 auf. Die Meßvorrichtung mit Meßaufnehmer ist mit 4 bezeichnet und der Ofen, der die Probe umgibt, mit 5. Alle diese Teile sind zwischen zwei Säulen 6 des Gestells 7 mit wenigstens einer Traverse 8 angeordnet und zwar so, daß die Spindel gekuppelt werden kann mit dem Zylinder der Dehnungsausgleichsvorrichtung und der Probenhalter mit einem zweiseitigen Hebel 9 an dem eine Belastungsmasse (Gewicht) 10 aufgehängt ist. Der zweiseitige Hebel weist ein Übersetzungsverhältnis von 20:1 auf. Er ist in Figur 2 nicht maßstäblich dargestellt.

In Figur 3a ist die Startposition der Dehnungsausgleichsvorrichtung für den Schalter mit der Kolbenstellung etwa in 1/2 Höhe dargestellt. Mit 14 ist die Kupplung für den Probenhalter 19a (Fig. 2) beziffert und mit 13 der Endschalter, der in dieser Position geschlossen ist. An Stelle eines mechanischen Kipphebelschalters kann auch eine Ausführung gewählt werden, die integrierte Kontakte und eine elektronische Kippschaltung aufweist.

In Figur 3 ist die gleiche Schalteinrichtung dargestellt, jedoch in ihrer Position vor Beginn des Versuchs. Beim Aufheizen kann sich der Versuchsaufbau, insbesondere die Probe 17 um das Maß A dehnen und der Probenhalter 19 a ist über Gestänge 15 am oberen Ende bei 8 mit dem zweiseitigen Hebel 9 verbunden und am unteren Ende über Zwischengestänge 15 und 19 mit dem Kolben 16 in einem Zylinder 11, der mit Hydrauliköl gefüllt ist. Hinter diesem befindet sich die Spindel 2, die zum Antriebsmotor im Maschinenbett bzw. Sockel 1 führt. Das Hysterese-Schaltgestänge 12 für 13 und 3 ist ebenfalls deutlich sichtbar (vgl. Fig. 1).

Wie aus Figur 3 c ersichtlich, ist nach Beendigung des Versuchs die Dehnungsausgleichseinrichtung um das Maß A kontrahiert, d. h. A ist ungefähr zu Null geworden.

Dabei ist der Kolben wie die Darstellung im Schnitt in Fig. 3 d deutlich macht, mit seiner Stange 18 in den Zylinder hineinbewegt worden, der durch sein Hydrauliköl die Bewegung dämpft bzw. sanft abfedert.

Dadurch wird ein Bruch der Halter und des Quarzstabs 19 vermieden, der den Kontakt zwischen Probe 17 und Aufnehmer 4 herstellt.

In Fig. 3 d ist ein Längsschnitt durch die hydraulische Dämpfungsvorrichtung gezeigt. Diese besteht im wesentlichen aus einem Zylinder 11, der von einem Deckel 39 verschlossen ist. Im Zylinder 11 ist ein Kolben 16 zwischen einem Minimalausdehnungsanschlag 31 und einem Maximalausdehnungsanschlag 38 bewegbar. Der Kolben 16 ist mittels eines Bolzens 40 mit der Kupplung 41 verbunden, die wiederum über ein in die Bohrung 14 a einsteckbaren Bolzen mit dem Gestänge 15 (siehe Fig. 2) der Materialprüfeinrichtung gekoppelt ist.

Der Deckel 39 weist eine Bohrung für die Durchführung einer hohlen Kolbenstange 18 auf, die zwischen Kolben 16 und Kupplung 41 eingespannt ist.

Im Zylinderraum 34 befindet sich Hydrauliköl, das bei Auftreten einer Druckkraft zwischen den Bohrungen 14 a und 14 b über die Durchflußbohrung 32 in den Hohlraum 33 innerhalb der Kolbenstange 18 fließen kann. Es ist eine Dichtung 42 im Deckel 39 vorgesehen. Der Endschalter 13 ist mit dem Zylinder 11 fest verschraubt. Das Hysterese-Schaltgestänge 12 ist oberhalb des Zylinders 11 gelegen.

Mit dem Oberteil fest verbunden ist die Schalterbetätigungsstange 12, die zwei zueinander beabstandete Stifte aufweist, so daß die Schalterbetätigung einen, dem Abstand A entsprechenden Hystereseweg aufweist. Am Oberteil ist die Kupplungseinrichtung 15 identisch mit der des Unterteils vorgesehen und dient dazu, bei 14 die Abkopplung an den Aufnehmer und den Probenhalter vorzunehmen.

Bei Bruch der Probe bewegt sich der Probenhalter, das Aufnehmerjoch und der Meßaufbau mit dem empfindlichen Dehnungsaufnehmer und den Quarzglasstäben in Richtung auf das Unterteil, wobei die Bewegung durch den Kolben 16 im Zylinder 11 infolge des darin enthaltenen Öls stark gedämpft wird. Dabei wird der Endschalter 13 betätigt, der über eine numerische Steuerung die Spindel 2 des Antriebsmotors und den Ofen ausschaltet.

Bei zeit- bzw. dehnungsgesteuerten Versuchen fährt bei Beendigung des Versuchs, gesteuert von der numerischen Steuerung, die Antriebsspindel 2 mit dem Unterteil in Richtung Oberteil, wobei nach Überwindung der Hysteresestrecke ebenfalls der Endschalter 13, die Ofenheizung und die Antriebsspindel abgeschalter wird. Da jedoch in diesem Betriebszustand noch eine feste Verbindung des Oberteils mit dem Belastungsgewicht 10 über dem Hebel 9 vorliegt - vorausgesetzt die Probe ist noch intakt - kontrahiert der gesamte Verzuchsaufbau, der sich im Ofenbereich befindet, infolge dessen abnehmender Temperatur. Jedoch steht jetzt der Hystereseweg A der Vorrichtung zur Verfügung, so daß die Kontraktion ohne Schaden des Versuchsaufbaus und der Probe erfolgen kann, d.h., während des Kontraktionsvorganges bleibt der gesamte Versuchsaufbau spannungslos.

Bevorzugt wird bei der erfindungsgemäßen Materialprüfvorrichtung ein Arbeiten mit einem Dehnungsaufnehmer nach dem Prinzip des inkrementalen Linearmeßsystems, d.h., der von der Probe übertragene Meßweg wirkt auf ein Quarzmeßelement auf welchem nach dem Photoresistverfahren eine Miniaturskala eingeätzt ist, die photooptisch abgetastet wird und ohne Umwandlung ein codiertes BCD-Signal ausgibt, welches direkt vom Prozeßrechner verarbeitet werden kann. Die beschriebene Materialprüfvorrichtung kann je nach Anwendungsfall entweder in einer vorgegebenen Zeit oder nach einer voreingestellten Dehnung vollautomatisch abschalten. Die gewünschten Prozeßparameter werden mittels Programm in den Prozeßrechner eingegeben.

Fig. 4 zeigt das Blockschaltbild der Hysterese- und Schalteinrichtung.

Wie aus Fig. 4 ersichtlich, werden im linken Teil I aus der Prüfvorrichtung die Istwerte 4, 22, 23 für den Computer C im Mittelteil gewonnen. Im rechten Teil O erfolgt die Ausgabe und ggf. Speicherung der Daten.

Mit 24 ist die Meßwertaufbereitung, z.B. Verstärkung und/oder A/D Umwandlung bezeichnet, mit 25 der Prozeßrechner z.B. ein HP 85 zur Meßwertverarbeitung. Er ist mit dem Sollwertgeber 28 (z.B. entsprechend den Istwerten) und dem Steuerteil 26 sowie dem Leistungssteller 27 verbunden.

Letzterer steuert die Heizung 21 für den Ofen 5 und den Antriebsmotor für die Spindel 2 (Fig. 1).

Die ausgegebenen Daten werden registriert z.B. in einem Plotter/Drucker 29 und gespeichert z.B. in einer Floppydisc 30.

Die erfindungsmäßige Materialprüfvorrichtung eignet sich gut zur serienmäßigen, 100%igen Kontrolle von Proben wie Werkstücken aus einer Serienfertigung, insbesondere von hochbelastbaren Teilen wie Schaufeln von Strömungsmaschinen.

## Patentansprüche

1. Materialprüfvorrichtung für Zeitstandsprüfungen an Probekörpern (17) aus metallischen oder keramischen Werkstoffen oder sich ähnlich verhaltenden Stoffen, bei der der Probekörper (17) beidendig eingespannt ist und unter erhöhter Temperatur belastbar ist, wobei ein Probenende mit einer Motorspindel (2) zum Zweck der Be-und Entlastung gekoppelt ist, und Dehnung oder Kontraktion mittels einer Meßvorrichtung mit Dehnungsaufnehmern (4) abfühlbar sind, dadurch gekennzeichnet, daß zwischen Motorspindel (2) und Probe (17) eine hydraulische Dehnungsausgleichsvorrichtung (3) mit einem in einem Hydraulikzylinder (11) gedämpft bewegbaren Kolben (16) vorhanden ist, wobei ein Schalter (13) derart zwischen Hydraulikzylinder (11) und Kolben (16) angeordnet ist, daß dieser bei maximal eingefahrenem Kolben (16) die Motorspindel (2) abschaltet.

2. Materialprüfvorrichtung nach Anspruch 1, dadurch gekennzeichnet, daß an der Dehnungsausgleichsvorrichtung (3) zwei zueinander um einen Hystereseweg des Kolbens (16) beabstandete Stifte (36, 37) angebracht sind, die mit dem Schalter (13) zum An- und Abschalten der Motorspindel (2) zusammenwirken.

## Claims

1. Material testing device for creep testing of test pieces (17) of metallic or ceramic materials or materials behaving in a similar manner, in which the test piece (17) is gripped at both ends and can be stressed at high temperature, in which one test piece end is coupled to a motor spindle (2) for the purpose of stressing and relieving stress, and expansion or contraction can be sensed by means of a measuring device with strain pickups (4), characterised in that between the motor spindle (2) and the test piece (17) there is a hydraulic expansion compensation device (3) with a plunger (16) movable with damping in a hydraulic cylinder (11), a switch (13) being arranged between the hydraulic cylinder (11) and the plunger (16) so that it switches off the motor spindle (2) when the plunger (16) has travelled in to its maximum extent.

2. Material testing device according to claim 1, characterised in that two pins (36, 37) are fitted to the expansion compensation device (3), spaced apart from each other by a hysteresis path of the plunger (16), and cooperate with the switch (13) for switching the motor spindle (2) on and off.

## Revendications

1. Dispositif d'essais de matériaux pour des essais de fluage d'éprouvette (17) en métaux ou matières céramiques ou matériaux analogues, dispositif selon lequel l'éprouvette (17) est fixée par ses deux extrémités et est soumise à une température élevée, une extrémité de l'éprouvette étant couplée à une broche de moteur (2) pour être soumise à une charge ou une décharge, et les allongements ou contractions sont captés par un dispositif de mesure comportant des capteurs d'allongement (4), dispositif caractérisé en ce qu'entre la broche (2) du moteur et l'éprouvette (17), il est prévu un dispositif de compensation hydraulique d'allongement (3) comprenant un cylindre hydraulique (11) recevant un piston (16) mobile de manière amortie, un commutateur (13) étant prévu entre le vérin hydraulique (11) et le piston (16) pour que lorsque le piston (16) est rentré au maximum, la broche (2) du moteur s'arrête.

2. Dispositif d'essais de matériaux selon la revendication 1, caractérisé en ce que le dispositif de compensation de dilatation (3) comporte deux broches (36, 37) écartées l'une de l'autre de la course d'hystérésis du piston (16), ces broches coopérant avec le commutateur (13) pour arrêter ou mettre en oeuvre la broche (2) du moteur.

FIG.1

FIG. 2

FIG.3a          FIG.3b          FIG.3c

EP 0 233 539 B1

FIG: 3d

FIG.4

EP 0 233 539 B1